# EUROPEAN PATENT APPLICATION

(11) **EP 4 831 164 A1**
(43) Date of publication of application: **28.10.2026**
(21) Application number: 24906037.7
(22) Date of filing: 29.11.2024
(51) Int. Cl.: C07D 305/12

(54) **METHOD FOR PREPARING SIZING AGENT**

(30) Priority: 21.12.2023 CN 202311776960
(71) Applicant: Kemira OYJ, 00180 Helsinki (FI)
(72) Inventor: JING, Rongkuan, Shanghai 201114 (CN); LIU, Chen, Shanghai 201114 (CN); LI, Can, Shanghai 201114 (CN); LI, Fengyang, Shanghai 201114 (CN)
(74) Representative: Cabinet Becker et Associés
(86) International application number: PCT/CN2024/135484
(87) International publication number: WO 2025/130552

(57) **Abstract**

The present invention relates to the field of papermaking. In particular, the present invention relates to a method for preparing a sizing agent. The method of the present invention comprises the steps of (1) pre-adding the first portion of TEA of total TEA to a HEEL-containing reaction system to obtain the HEEL-containing reaction system with pre-added TEA; and (2) adding the second portion of TEA of the total TEA and FAC to the HEEL-containing reaction system with pre-added TEA to carry out the reaction, wherein the weight percent of the first portion of the total TEA is no greater than 90% of the total TEA. The method of the present invention can reduce the viscosity of the reaction system and/or enable toluene-free operation.

## Description

### TECHNICAL FIELD

The present invention relates to the field of papermaking. In particular, the present invention relates to a method for preparing a sizing agent. The method of the present invention can reduce the viscosity of the reaction system and/or enable toluene-free operation.

### BACKGROUND

Paper is formed by intertwining of materials containing plant cellulose. The plant cellulose has hydroxyl groups, which impart the paper a certain degree of hydrophilicity. The plant cellulose is also intertwined to form pore structures, which have capillary action, and impart the paper a certain degree of permeability of fluids (e.g., water, oils, inks, juices, etc.). Therefore, the paper surface often requires treatment to improve performance. During the papermaking process, the paper surface can be treated by applying a sizing agent. Depending on the pH value, sizing agents can be classified as acidic sizing agents, neutral sizing agents and alkaline sizing agents. In recent years, neutral/alkaline sizing papermaking has gained increasing interest in the papermaking industry.

A commonly used neutral sizing agent is Alkyl Ketene Dimer (AKD), which has been used for more than 50 years. AKD is a waxy solid at room temperature. AKD can be used both as an internal sizing agent and a surface sizing agent. AKD is usually represented by the following structure: 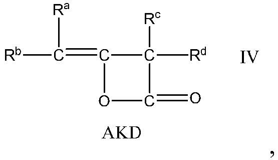 wherein each of R^{a}, R^{b}, R^{c} and R^{d} groups for each occurrence is independently selected from: hydrogen; optionally substituted, unbranched or branched C₁-C₃₀ hydrocarbon groups. It has been found that each of the R^{a}, R^{b}, R^{c} and R^{d} groups in the above ketene dimer may be a saturated hydrocarbon group, or an unsaturated hydrocarbon group (e.g., an alkenyl derived from oleic acid). By convention, ketene dimers with unsaturated hydrocarbon groups are also referred to as AKD (alkyl ketene dimers).

The four-membered lactone ring in the AKD molecule can react with the hydroxyl group on the cellulose to form an ester bond, thereby being attached to the cellulose. The long-chain hydrophobic groups of AKD (i.e., one or more of R^{a}, R^{b}, R^{c}, and R^{d}) produce hydrophobic effects on the fiber surface and cover part of the pore structure, thereby imparting hydrophobicity and permeation resistance to the paper. The reaction of AKD with cellulose is shown schematically as follows:

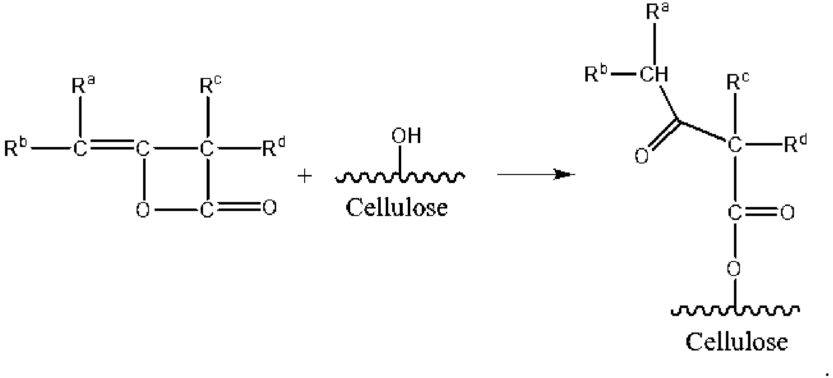

A typical method for preparing AKD comprises the step of adding fatty acid chlorides (FACl) and triethylamine (TEA) to a reactor. During the above preparation process, the viscosity in said reaction system increases with the formation of the by-product triethylamine hydrochloride (TEA·HCl), which leads to a number of problems such as difficulty in stirring, increased energy consumption, and poor heat dissipation. In many cases, it often requires the addition of inert solvents such as toluene to reduce the adverse effects of high viscosity in industry. With the increase in environmental awareness, there is also a further requirement to reduce the use of toluene.

A known method for reducing viscosity is to feed the residues from the previous batch for sizing agent production (also referred to as HEEL, which comprises AKD and TEA·HCl, and optionally unreacted TEA,) into the production of the next batch. The HEEL is beneficial to reduce the viscosity of the reaction system of that batch, but it still cannot completely eliminate the use of toluene.

At present, there is a continuing need in this field for preparation methods of sizing agents (especially AKD) that can be performed in a reduced viscosity, as well as toluene-free.

### SUMMARY OF THE INVENTION

Methods for preparing AKDs are well known in the art. For example, AKDs are usually produced, with carboxylic acids as raw materials, through three steps: acylation, dehydrochlorination and dimerization. Exemplary synthetic steps for AKDs are as shown below.

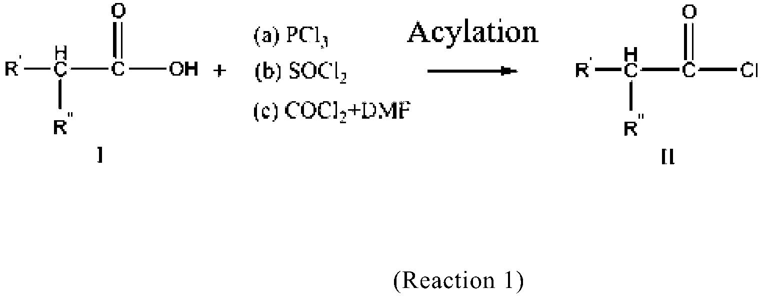

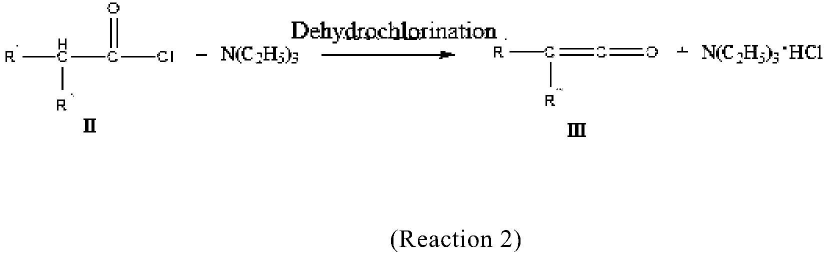

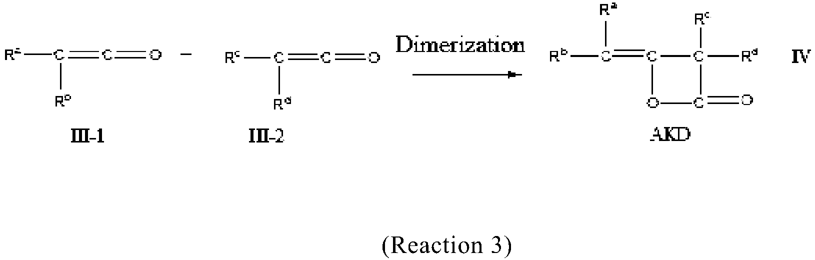

In the AKD shown in structural formula (IV),
R^{a} group for each occurrence is independently selected from: hydrogen; optionally substituted, unbranched or branched C₁-C₃₀ hydrocarbon group;
R^{b} group for each occurrence is independently selected from: hydrogen; optionally substituted, unbranched or branched C₁-C₃₀ hydrocarbon group;
R^{c} group for each occurrence is independently selected from: hydrogen; optionally substituted, unbranched or branched C₁-C₃₀ hydrocarbon group; and,
R^{d} group for each occurrence is independently selected from: hydrogen; optionally substituted, unbranched or branched C₁-C₃₀ hydrocarbon group.

With regard to any of R^{a}, R^{b}, R^{c} or R^{d}, the C₁-C₃₀ hydrocarbon group is preferably a C₁₀-C₂₀ hydrocarbon group, and more preferably a C₁₃-C₁₇ hydrocarbon group, such as C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀ hydrocarbon group. With regard to any of R^{a}, R^{b}, R^{c} or R^{d}, the hydrocarbon group may be alkyl, alkenyl, or alkynyl; preferably alkyl.

In any one of structural formulae I, II and/or III, the R' group for each occurrence is independently selected from: hydrogen; optionally substituted, unbranched or branched C₁-C₃₀ hydrocarbon groups; and/or the R" group for each occurrence is independently selected from: hydrogen; optionally substituted, unbranched or branched C₁-C₃₀ hydrocarbon groups.

With regard to any of R' or R", the C₁-C₃₀ hydrocarbon group is preferably a C₁₀-C₂₀ hydrocarbon group, and more preferably a C₁₃-C₁₇ hydrocarbon group, such as C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀ hydrocarbon group. With regard to any of R' or R", the hydrocarbon group may be alkyl, alkenyl, or alkynyl; preferably alkyl.

Ketene of formula (III-1) 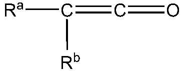 is synthesized using the same steps as ketene of formula (III-2) 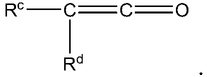 The R^{a} group for each occurrence is independently selected from: hydrogen; optionally substituted, unbranched or branched C₁-C₃₀ hydrocarbon group; the R^{b} group for each occurrence is independently selected from: hydrogen; optionally substituted, unbranched or branched C₁-C₃₀ hydrocarbon group; the R^{c} group for each occurrence is independently selected from: hydrogen; optionally substituted, unbranched or branched C₁-C₃₀ hydrocarbon group; and the R^{d} group for each occurrence is independently selected from: hydrogen; optionally substituted, unbranched or branched C₁-C₃₀ hydrocarbon group. With regard to any of R^{a}, R^{b}, R^{c} or R^{d}, the C₁-C₃₀ hydrocarbon group is preferably a C₁₀-C₂₀ hydrocarbon group, and more preferably a C₁₃-C₁₇ hydrocarbon group, such as C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀ hydrocarbon group. With regard to any of R^{a}, R^{b}, R^{c} or R^{d}, the hydrocarbon group may be alkyl, alkenyl, or alkynyl; preferably alkyl.

In the acylation step, (a) PCl₃, (b) SOCl₂ and (c) COCl₂ +DMF are three commonly used acylation methods. Method (c) using COCl₂ and DMF is currently the most commonly used acylation method, where phosgene (COCl₂) is used as the acylating agent and dimethylformamide (DMF) is used as the catalyst.

In the dehydrochlorination step, triethylamine hydrochloride (N(CH₂CH₃)₃·HCl) is usually recycled after regeneration to triethylamine for cost-saving.

In reaction 2 above, the raw material of formula (II) is commonly referred to as fatty acid chlorides (FACl), which undergoes dehydrochlorination under the action of triethylamine (TEA). Correspondingly, only for the purpose of ease in depiction, the raw material of formula (I) in the above reaction 1 is herein described as fatty acid (FA), no matter whether it is saturated or unsaturated.

Therefore, a typical step of the AKD preparation method involves contacting TEA and FACl to react, and the reaction equation can be as follows. $2 FACl + 2 TEA \to AKD + 2 TEA ⋅ HCl .$

The inventor of the present invention surprisingly found that by pre-adding a first portion of TEA of total TEA to a HEEL-containing reaction system to obtain the HEEL-containing reaction system with the pre-added TEA, followed by adding a remaining second portion of TEA of the total TEA and FACl to the HEEL-containing reaction system with the pre-added TEA to carry out the reaction, the viscosity of the reaction system can be significantly reduced, which allows for solvent-free (e.g., toluene-free) during the preparation of AKDs.

### BRIEF DESCRIPTION OF THE DRAWINGS

Figure 1 shows the viscosity of reaction system for Examples 1-4.
Figure 2 shows the viscosity of reaction system for Examples 6-8.

### DETAILED DESCRIPTION OF EMBODIMENTS

In some embodiments of the invention, the present invention provides a method for preparing a sizing agent, comprising the steps of:
(1) pre-adding a first portion of TEA of total TEA to HEEL to obtain the HEEL-containing reaction system with the pre-added TEA; and
(2) adding a remaining second portion of TEA of the total TEA and FACl to the HEEL-containing reaction system with the pre-added TEA to carry out the reaction;
wherein the weight percent of the first portion of TEA is no greater than 90 % of the total TEA.The weight percent of the first portion of TEA of the total TEA is 100% × the ratio of the weight of the first portion of TEA to the sum of the weight of the first portion of TEA and the weight of the remaining second portion of TEA.

In one aspect of the invention, the HEEL-containing reaction system of the present invention comprises any materials/intermediates/products involved in the production of said sizing agent, such as FA, TEA, FACl, alkyl ketene, AKD, TEA·HCl, or a combination thereof. In a preferred embodiment, the HEEL-containing reaction system comprises FACl, TEA, AKD, TEA·HCl, or a combination thereof.

In one embodiment, the HEEL described herein refers to any mixtures comprising AKD and TEA·HCl. Optionally, said HEEL further comprises TEA or other components. The HEEL described herein could be fresh or recycled. In one embodiment, the HEEL described herein is a portion of the reaction mixture of the previous batch for sizing agent production, which comprises AKD and TEA·HCl, preferably further comprises unreacted TEA.

In one embodiment, the AKD and TEA·HCl in the HEEL may be present in any ratio. In one embodiment, said AKD and TEA·HCl are in a weight ratio of 1:1 to 10:1, preferably 3:1 to 6:1.

In another embodiment, the AKD, TEA·HCl and TEA (if any) may be present in any ratio. In still another embodiment, said AKD, TEA·HCl and TEA (if any) are in a weight ratio of 1:1:5 to 10:1:1, preferably 3:1:1 to 6:1:1.

In one aspect of the invention, the reaction mixture of the present invention comprises FA, TEA, FACl, alkyl ketene, AKD, TEA·HCl, or a combination thereof. In one embodiment, the reaction mixture described herein refers to the mixture in the following reaction: (2 FACl + 2 TEA → AKD + 2 TEA·HCl).

In one embodiment, in addition to HEEL, the HEEL-containing reaction system described herein further comprises FA, TEA, FACl, alkyl ketene, AKD, TEA·HCl, or a combination thereof. In another embodiment, the HEEL-containing reaction system described herein comprises nothing but HEEL.

Herein, any materials, such as FA, TEA, FACl, alkyl ketene, AKD, and/or, TEA·HCl could be fresh or recycled.

In one embodiment, the fatty acyl chloride (FACl) described herein is a compound having structural formula (II): 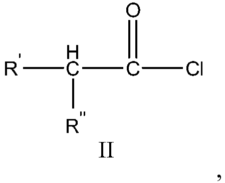 wherein,
R' group for each occurrence is independently selected from: hydrogen; optionally substituted, unbranched or branched C₁-C₃₀ hydrocarbon groups; and/or
R" group for each occurrence is independently selected from: hydrogen; optionally substituted, unbranched or branched C₁-C₃₀ hydrocarbon groups.

With regard to any of R' or R", the C₁-C₃₀ hydrocarbon group is preferably a C₁₀-C₂₀ hydrocarbon group, more preferably a C₁₃-C₁₇ hydrocarbon group, such as C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀ hydrocarbon group. With regard to any of R' or R", the hydrocarbon group may be alkyl, alkenyl, or alkynyl; preferably alkyl.

In one aspect of the invention, the fatty acyl chloride (FACl) described herein can be a mixture of two or more fatty acyl chlorides. Similarly, the AKDs described herein may be a mixture of two or more AKDs. The fatty acid (FA) described herein can be a mixture of two or more fatty acids. In one embodiment, the FACl, FA, and AKD described herein can have the same or different hydrocarbon moieties.

In one aspect of the invention, the additions of FACl and the second portion of TEA (preferably added simultaneously) are carried out at a temperature between 50°C and 70°C, e.g., 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C; preferably between 52°C and 68°C; more preferably between 52°C and 65°C.

In one aspect of the present invention, the weight percent of the first portion of TEA of the total TEA is no greater than 90 % of the total TEA, for example, ranging from greater than 0% to less than 90%, such as 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%; or ranging between any two of the aforementioned values, preferably between 10% and 90%.

In a preferred embodiment, the additions of FACl and the second portion of TEA (preferably added simultaneously) are carried out at a temperature between 52°C and 68°C.

In one aspect of the invention, the weight percent of the first portion of TEA of the total TEA ranges from 10% to 90%. In a preferred embodiment, the additions of FACl and the second portion of TEA (preferably added simultaneously) in step (2) are carried out at a temperature between 52°C and 68°C, more preferably between 52°C and 65°C.

In one aspect of the invention, FACl and/or the second portion of TEA is added at the same or different rates in step (2). In some embodiments, the FACl is added at a rate of from 5 ml/min to 20 ml/min, preferably the rate is from 6 ml/min to 16 ml/min. In some embodiments, the TEA is added at a rate of from 1 ml/min to 10 ml /min, preferably the rate is 4 ml/min to 8 ml/min.

In one aspect of the invention, the viscosity of the reaction system is less than 250 cp, less than 240 cp, less than 230 cp, less than 220 cp, less than 210 cp, less than 200 cp, less than 190 cp, less than 180 cp, less than 170 cp, less than 160 cp, less than 150 cp, less than 140 cp, less than 130 cp, less than 120 cp, less than 110 cp, less than 100 cp or less than 90 cp throughout the reaction process.

In one aspect of the invention, the viscosity of the reaction system is less than 250 cp, less than 240 cp, less than 230 cp, less than 220 cp, less than 210 cp, less than 200 cp, less than 190 cp, less than 180 cp, less than 170 cp, less than 160 cp, less than 150 cp, less than 140 cp, less than 130 cp, less than 120 cp, less than 110 cp, less than 100 cp or less than 90 cp when the reaction is terminated.

In one aspect of the invention, the ratio of the total moles of the first portion of TEA and the second portion of TEA to the moles of FACl is 0.95:1 to 2:1, preferably 1.4:1 to 1.75:1. In one aspect of the invention, the ratio of the total weight of the first portion of TEA and the second portion of TEA to the weight of FACl is 1:3 to 2:3, preferably 1:1.7 to 1:2.1.

The method described herein can reduce the viscosity of the reaction mixture. The reduced viscosity of the reaction mixture results in a reduction of the energy consumption required for mixing. At the same time, the reduced viscosity of the reaction mixture enables the production of sizing agents (e.g., AKDs) under solvent-free (e.g., toluene-free) conditions/operations.

In one aspect of the present invention, the present invention relates to a method for preparing a sizing agent under toluene-free conditions, comprising the steps of:
(1) pre-adding the first portion of TEA of total TEA to a HEEL-containing reaction system to obtain the HEEL-containing reaction system with the pre-added TEA; and
(2) adding a second remaining portion of TEA of total TEA and FACl to the HEEL-containing reaction system with the pre-added TEA to carry out the reaction;
   the weight percent of the first portion of TEA of the total TEA is no greater than 90% of the total TEA.

In one aspect of the present invention, the present invention relates to a method for preparing a sizing agent with a reduced viscosity (e.g., a reduced viscosity of the reaction mixture) during preparation process, comprising the steps of:
(1) pre-adding the first portion of TEA to a HEEL-containing reaction system to obtain a HEEL-containing reaction system with the pre-added TEA; and
(2) adding a second remaining portion of TEA and FACl to the HEEL-containing reaction system with the pre-added TEA to carry out the reaction;
   a weight percent of the first portion of TEA of the total TEA is no greater than 90% of the total TEA.

As used herein, the term "substituted" means that any one or more hydrogens on the specified atom or group are replaced by a moiety selected from the designated groups, provided that the normal valence of the specified atom is not exceeded.

"Alkyl" is a branched or unbranched, saturated aliphatic hydrocarbon group. In one embodiment, the alkyl group contains from 1 to about 20 carbon atoms, more generally from 1 to about 12 carbon atoms, from 1 to about 6 carbon atoms, or from 1 to about 4 carbon atoms. In one embodiment, the alkyl group contains from 1 to about 8 carbon atoms. In some embodiments, the alkyl group is C₁-C₂, C₁-C₃ or C₁-C₆. As used herein, the designated range means that each member of the range is a separate species of alkyl group. For example, as used herein, the term C₁-C₃₀ alkyl refers to an unbranched or branched alkyl group with 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 carbon atoms and means that each of these is a separate species. For example, as used herein, the term C₁-C₄ alkyl refers to unbranched or branched alkyl groups with 1, 2, 3, or 4 carbon atoms and means that each of these is a separate species. When C₀-Cₙ alkyl is used herein in combination with another group, e.g., (C₃-C₇ cycloalkyl)C₀-C₄ alkyl or - C₀-C₄ alkyl(C₃-C₇ cycloalkyl), the designated group - in this case cycloalkyl - is either bonded directly by a single covalent bond (C₀ alkyl) or connected via an alkyl chain (in this case, of 1, 2, 3 or 4 carbon atoms). Alkyl groups may also be linked via other groups such as heteroatoms, as in -O-C₀-C₄ alkyl (C₃-C₇ cycloalkyl). Examples of alkyl include, but are not limited to, methyl, ethyl, n-propyl, isopropyl, n-butyl, isobutyl, sec-butyl, tert-butyl, n-pentyl, iso-pentyl, tert-pentyl, neopentyl, n-hexyl, 2-methylpentyl, 3-methylpentyl, 2,2-dimethylbutyl and 2,3-dimethylbutyl. In one embodiment, the alkyl group is optionally substituted as described above.

"Alkenyl" is a branched or unbranched, aliphatic hydrocarbon moiety with one or more carbon-carbon double bond(s), wherein said double bond(s) may occur at a stable position along the chain. Non-limiting examples include C₂-C₈ alkenyl, C₂-C₆ alkenyl, and C₂-C₄ alkenyl. As used herein, a designated range means that each member of said range is a separate species of alkenyl group, as described above for alkyl. For example, as used herein, the term C₁-C₃₀ alkenyl refers to an unbranched or branched alkenyl group with 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 carbon atoms and means that each of these is a separate species. Examples of alkenyl groups include, but are not limited to, vinyl and propenyl. In one embodiment, the alkenyl group is optionally substituted as described above.

"Alkynyl" is a branched or unbranched, aliphatic hydrocarbon moiety with one or more carbon-carbon triple bond(s), wherein said triple bond(s) may occur at a stable position along the chain, e.g., C₂-C₈ alkynyl or C₂-C₆ alkynyl. As used herein, a designated range means that each member of said range is a separate species of alkynyl group, as described above for alkyl. For example, as used herein, the term C₁-C₃₀ alkynyl refers to an unbranched or branched alkenyl group with 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 carbon atoms and means that each of these is a separate species. Examples of alkynyl groups include, but are not limited to, ethynyl, propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, and 5-hexynyl. In one embodiment, the alkynyl group is optionally substituted as described above.

"Hydrocarbyl" is a branched or unbranched, saturated or unsaturated aliphatic group. The hydrocarbon group may be alkyl, alkenyl, or alkynyl. As used herein, the designation range means that each member of said range is a separate group. For example, as used herein, the term C₁-C₃₀ hydrocarbyl refers to a hydrocarbyl group with 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 carbon atoms and means that each of these is a separate species.

The term "temperature" described herein is in degrees Celsius (°C). The concentration/content/amount/parts described herein may be a concentration/content/amount/parts expressed in weight, volume, moles, weight/volume, volume/weight, as required.

The term "addition" or "added" described herein are those performed by any means. The second portion of TEA and FACl described herein may be added simultaneously or separately. The second portion of TEA and FACl described herein may be added at the same rate or at different rates.

Herein, the sum of the first portion of TEA and the (remaining) second portion of TEA can be referred to as total TEA. Similarly, the sum of the weight of the first portion of TEA and the weight of the (remaining) second portion of TEA can be referred to as the weight of total TEA.

The total TEA described herein refers to the full amount of TEA to be used, such as the total amount of TEA to be used, calculated based on FACl. The amount may be insufficient, equal or excessive compared to that of FACl. Those skilled in the art could determine a reasonable total amount of TEA, since the preparation of AKD by reacting TEA with FACl is known to those skilled in the art. Generally, those skilled in the art adopt weight as the unit for feeding and monitoring in this reaction, as said reaction actually is not conducted in a stoichiometric manner.

Herein, the weight percent of the first portion of TEA is 100% × the ratio of the weight of the first portion of TEA to the sum of the weight of the first portion of TEA and the weight of the remaining second portion of TEA and can be referred to as X value or X range.

For example, in the present invention, the HEEL may be used in 5 to 40 wt%, preferably between 5 to 20 wt%, of the amount of the reaction mixture of the previous batch for sizing agent production. In one embodiment, the amount of HEEL accounts for 5 to 40 wt% of the amount of total reaction system.

The method described herein may be carried out in a continuous process or in a batch process, preferably in a batch process.

Herein, the terms "reaction mixture" and "reaction system" can be used interchangeably.

Herein, the term "a first portion of TEA" refers to the portion of TEA intended to be pre-added to the HEEL-containing reaction system.

The technical solution of the present invention will now be described with reference to specific examples. It should be understood that the following examples are used only to illustrate the invention and are not intended to specify the scope of the invention. Unless otherwise indicated, the reagents described in these examples are commercially available.

### Examples

### Example 1 (Control Group): No first portion of TEA

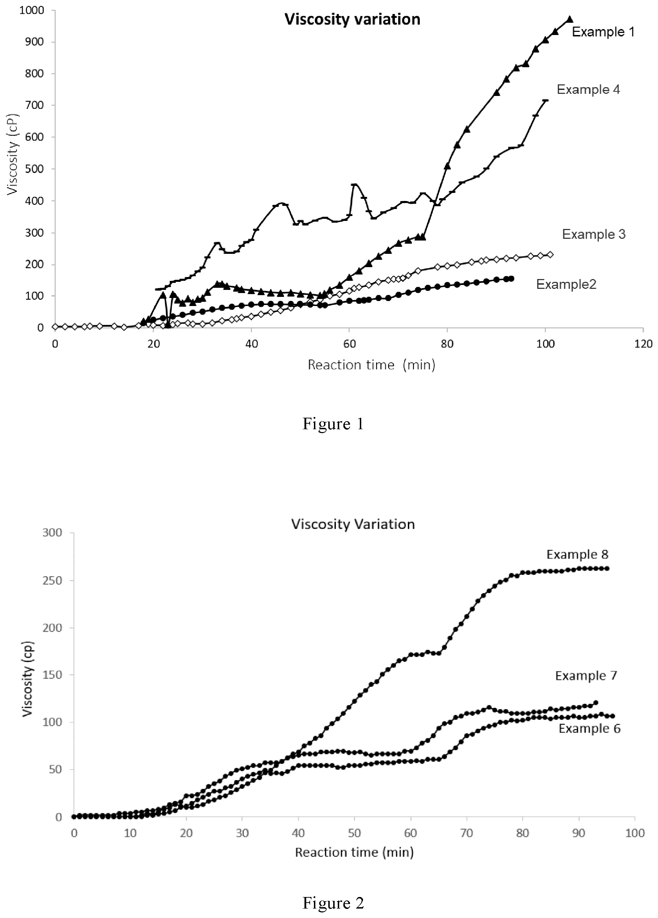

Before feeding, the reactor was evacuated and purged with nitrogen 2-3 times. 40.6 g AKD wax powder (n-C16 alkyl AKD: n-C18 alkyl AKD =60:40 (wt%), Kemira, FennoWax 1840 TF) was put into the reactor, then stirred, heated till molten, and added 10.8 g triethylamine hydrochloride (99%, Shanghai Macklin Biochemical Technology Co., Ltd.). After mixing well, 405 g of acyl chloride (n-C16 acyl chloride:n-C18 acyl chloride=60:40 (wt%), Kemira) was added dropwise at a rate of 13.8 ml/min. At the same time, 219 g of triethylamine was added dropwise at a rate of 6.8 ml/min, and the temperature of the system was kept at 60-62°C during the dropwise addition. After the dropwise addition was completed, the temperature was kept at 60°C for 1 hour. The viscosity of the reaction system was monitored by the Hydramotion online viscometer throughout the reaction process (Fig. 1), and the final viscosity of the system was 973 cP. Separation was conducted to yield pale yellow AKD product, with a purity of greater than 85% (in conformity with the executive standard GB/T 27565-2011).

### Example 2: the weight percent of the first portion of TEA of the total TEA is 70%

Before feeding, the reactor was evacuated and purged with nitrogen 2-3 times. 40.6 g AKD wax powder (n-C16 alkyl AKD: n-C18 alkyl AKD =60:40 (wt%), Kemira, FennoWax 1840 TF) was put into the reactor, then stirred, heated till molten, and added 10.8 g, triethylamine hydrochloride (99%, Shanghai Macklin Biochemical Technology Co., Ltd.) and 153 g triethylamine (analytically pure, Shanghai Lingfeng Chemical Reagent Co. Ltd.). After mixing well, 400 g of acyl chloride (n-C16 acyl chloride:n-C18 acyl chloride=60:40 (wt%), Kemira) was added dropwise at a rate of 14.2 ml/min. At the same time, 66 g of triethylamine was added dropwise at a rate of 5.2 ml/min, and the temperature of the system was kept at 60-62°C during the dropwise addition. After the dropwise addition was completed, the temperature was kept at 60°C for 1 hour. The viscosity of the reaction system was monitored by the Hydramotion online viscometer throughout the reaction process (Fig. 1), and the final viscosity of the system was 155.1 cP. Separation was conducted to yield pale yellow AKD product, with a purity of greater than 85% (in conformity with the executive standard GB/T 27565-2011).

### Example 3: the weight percent of the first portion of TEA of the total TEA is 6.4%

Before feeding, the reactor was evacuated and purged with nitrogen 2-3 times. 40.6 g AKD wax powder (n-C16 alkyl AKD: n-C18 alkyl AKD =60:40 (wt%), Kemira, FennoWax 1840 TF) was put into the reactor, then stirred, heated till molten, and added 10.8 g triethylamine hydrochloride (99%, Shanghai Macklin Biochemical Technology Co., Ltd.) and 14 g triethylamine (analytically pure, Shanghai Lingfeng Chemical Reagent Co. Ltd.). After mixing well, 400 g of acyl chloride (n-C16 acyl chloride:n-C18 acyl chloride=60:40 (wt%), Kemira) and 205 g of triethylamine was added dropwise. The temperature of the system was kept at 60-62°C during the dropwise addition. After the dropwise addition was completed, the temperature was kept at 60°C for 1 hour. The viscosity of the reaction system was monitored by the Hydramotion online viscometer throughout the reaction process (Fig. 1), and the final viscosity of the system was 228.3 cP. Separation was conducted to yield pale yellow AKD product, with a purity of greater than 85% (in conformity with the executive standard GB/T 27565-2011).

### Example 4 (Control Group): the weight percent of the first portion of TEA of the total TEA is 100%

Before feeding, the reactor was evacuated and purged with nitrogen 2-3 times. 40.6 g AKD wax powder (n-C16 alkyl AKD: n-C18 alkyl AKD =60:40 (wt%), Kemira, FennoWax 1840 TF) was put into the reactor, then stirred, heated till molten, and added 10.8 g triethylamine hydrochloride (99%, Shanghai Macklin Biochemical Technology Co., Ltd.) and 219 g triethylamine (analytically pure, Shanghai Lingfeng Chemical Reagent Co. Ltd.). After mixing well, 400 g of acyl chloride (n-C16 acyl chloride:n-C18 acyl chloride=60:40 (wt%), Kemira) was added dropwise. The temperature of the system was kept at 60-62°C during the dropwise addition. After the dropwise addition was completed, the temperature was kept at 60°C for 1 hour. The viscosity of the reaction system was monitored by the Hydramotion online viscometer throughout the reaction process (Fig. 1), and the final viscosity of the system was 715 cP. Separation was conducted to yield pale yellow AKD product, with a purity of greater than 85% (in conformity with the executive standard GB/T 27565-2011).

### Example 5: the weight percent of the first portion of TEA of the total TEA (146.1 g) is 1.4%

Before feeding, the reactor was evacuated and purged with nitrogen 2-3 times. 40.6 g AKD wax powder (n-C16 alkyl AKD: n-C18 alkyl AKD =5:95 (wt%), Kemira, FennoWax 1895 TFE) was put into the reactor, then stirred, heated till molten, and added 10.8 g triethylamine hydrochloride (99%, Shanghai Macklin Biochemical Technology Co., Ltd.) and 2.1 g triethylamine (analytically pure, Shanghai Lingfeng Chemical Reagent Co. Ltd.). After mixing well, the system temperature was lowered to about 58°C, the mixture solidified, stirring was difficult, and the reaction could not proceed at this temperature.

### Example 6: the weight percent of the first portion of TEA of the total TEA is 21%

Before feeding, the reactor was evacuated and purged with nitrogen 2-3 times. 69.4 g AKD wax powder (n-C16 alkyl AKD: n-C18 alkyl AKD =5:95 (wt%), Kemira, FennoWax 1895 TFE) was put into the reactor, then stirred, heated till molten, and added 35.3 g triethylamine hydrochloride (99%, Shanghai Macklin Biochemical Technology Co., Ltd.) and 30.8 g triethylamine (analytically pure, Shanghai Lingfeng Chemical Reagent Co. Ltd.). After mixing well, 405 g of acyl chloride (n-C16 acyl chloride:n-C18 acyl chloride=5:95 (wt%), Kemira) and 115.3 g triethylamine were added dropwise. The temperature of the system was kept at 58-60°C during the dropwise addition. After the dropwise addition was completed, the temperature was kept at 63°C for half an hour. The viscosity of the reaction system was monitored by the Hydramotion online viscometer throughout the reaction process (Fig. 2), and the final viscosity of the system was 106.6 cP. Separation was conducted to yield pale yellow AKD product, with a purity of greater than 88% (in conformity with the executive standard GB/T 27565-2011).

### Example 7: the weight percent of the first portion of TEA of the total TEA is 21%

Before feeding, the reactor was evacuated and purged with nitrogen 2-3 times. 69.4 g AKD wax powder (n-C16 alkyl AKD: n-C18 alkyl AKD =5:95 (wt%), Kemira, FennoWax 1895 TFE) was put into the reactor, then stirred, heated till molten, and added 35.3 g triethylamine hydrochloride (99%, Shanghai Macklin Biochemical Technology Co., Ltd.) and 30.8 g triethylamine (analytically pure, Shanghai Lingfeng Chemical Reagent Co. Ltd.). After mixing well, 405 g of acyl chloride (n-C16 acyl chloride:n-C18 acyl chloride=5:95 (wt%), Kemira) and 115.3 g triethylamine were added dropwise. The temperature of the system was kept at 62-64°C during the dropwise addition. After the dropwise addition was completed, the temperature was kept at 63°C for half an hour. The viscosity of the reaction system was monitored by the Hydramotion online viscometer throughout the reaction process (Fig. 2), and the final viscosity of the system was 120.5 cP. Separation was conducted to yield pale yellow AKD product, with a purity of greater than 88% (in conformity with the executive standard GB/T 27565-2011).

### Example 8 (Control Group): the weight percent of the first portion of TEA of the total TEA is 100%

Before feeding, the reactor was evacuated and purged with nitrogen 2-3 times. 69.4 g AKD wax powder (n-C16 alkyl AKD: n-C18 alkyl AKD =5:95 (wt%), Kemira, FennoWax 1895 TFE) was put into the reactor, then stirred, heated till molten, and added 35.3 g triethylamine hydrochloride (99%, Shanghai Macklin Biochemical Technology Co., Ltd.) and 146.1 g triethylamine (analytically pure, Shanghai Lingfeng Chemical Reagent Co. Ltd.). After mixing well, 405 g of acyl chloride (n-C16 acyl chloride:n-C18 acyl chloride=5:95 (wt%), Kemira) was added dropwise. The temperature of the system was kept at 62-64°C during the dropwise addition. After the dropwise addition was completed, the temperature was kept at 63°C for half an hour. The viscosity of the reaction system was monitored by the Hydramotion online viscometer throughout the reaction process (Fig. 2), and the final viscosity of the system was 262.8 cP. Separation was conducted to yield pale yellow AKD product, with a purity of greater than 88% (in conformity with the executive standard GB/T 27565-2011).

The mechanisms described in the specification of the present application are intended only to explain the invention and are not intended to constitute a limitation of the invention in any way.

The above descriptions of examples, embodiments and implementations should be regarded as illustrative and not as limiting the invention described herein. The above specific examples are only specific embodiments of the present invention, and the protection scope of the present invention includes, but is not limited to, the embodiments of the above-mentioned specific examples. Any embodiments conforming to the scope of the present invention as recorded herein and any appropriate changes or modifications made by those skilled in the art, shall fall within the protection scope of the present invention.

As used herein, the terms "approximate", "about", "substantially", and the like are intended to have a broad meaning that is generally acknowledged and accepted by those skilled in the art concerning the subject matter of this disclosure. It will be understood by those skilled in the art, after reading this disclosure that these terms are intended to the description of certain features described and claimed, without limiting these features to the precise numerical ranges provided. Accordingly, these terms are to be read as indicating that non-substantial or inessential amendments or variations to the subject matter described and claimed are deemed to be within the scope of the invention as recited in the appended claims.

As used herein, the words "optional" or "optionally" are intended to indicate that the subsequently described event or circumstance may be present or absent, and that the description includes instances in which the stated event or circumstance occurs and instances in which it does not. Unless otherwise stated, the indefinite article "a" or "an" and its corresponding definite article "the" or "said" used herein mean to at least one, or one or more.

## Claims

1. A method for preparing a sizing agent, comprising the steps of:
(1) pre-adding the first portion of TEA of total TEA to a HEEL-containing reaction system to obtain the HEEL-containing reaction system with the pre-added TEA; and
(2) adding a remaining second portion of TEA of the total TEA and FACl to the HEEL-containing reaction system with the pre-added TEA to carry out the reaction;
wherein the weight percent of the first portion of the total TEA is no greater than 90% of the total TEA.

2. The method according to claim 1, wherein the addition of the second portion of TEA and FACl in step (2) is carried out at a temperature between 50°C and 70°C, preferably between 52°C and 68°C.

3. The method according to claim 1 or 2, wherein the weight percent of the first portion of the total TEA is between 10% to 90% of the total TEA.

4. The method according to any one of the preceding claims, wherein the addition of the second portion of TEA and FACl in step (2) is carried out at a temperature between 52°C and 68°C, and the weight percent of the first portion of the first portion of the total TEA ranges from 10% to 90% of the total TEA.

5. The method according to any one of the preceding claims, wherein FACl and the second portion of TEA are added at the same or different rates in step (2).

6. The method according to claim 5, wherein the rate of the addition of FACl is from 5 ml/min to 20 ml/min, preferably the rate is from 6 ml/min to 16 ml/min.

7. The method according to claim 5, wherein the second portion of TEA is added at a rate of 1 ml/min to 10 ml/min, preferably the rate is 4 ml/min to 8 ml/min.

8. The method according to any one of the preceding claims, wherein the ratio of the total weight of the first portion of TEA and the second portion of TEA to the weight of FACl is 1:3 to 2:3, preferably 1:1.7 to 1:2.1.

9. The method according to any one of the preceding claims, wherein, in addition to HEEL, the HEEL-containing reaction system further comprises FA, TEA, FACl, alkyl ketene, AKD, TEA·HCl, or a combination thereof.

10. The method according to any one of the preceding claims, wherein the sizing agent is an alkyl ketene dimer (AKD).

11. The method according to any one of the preceding claims, wherein the viscosity of the reaction system is less than 250 cp, preferably less than 200 cp, more preferably less than 150 cp, most preferably less than 100 cp, throughout the reaction process.

12. The method according to any one of the preceding claims, wherein the viscosity of the reaction system is less than 250 cp, preferably less than 200 cp, more preferably less than 150 cp, most preferably less than 100 cp, when the reaction is terminated.
